# FASCICULE DE BREVET EUROPEEN

(11) **EP 2 210 580 B1**
(45) Date de publication et mention de la délivrance du brevet: **06.03.2019**
(21) Numéro de dépôt: 09151476.0
(22) Date de dépôt: 27.01.2009
(51) Int. Cl.: A61H 35/04, A61M 3/02

(54) **Canule pour l'administration d'une solution fluide dans une narine**
Kanüle zur Verabreichung einer flüssigen Lösung in ein Nasenloch
Nozzle for the administration of a liquid solution in a nostril

(43) Date de publication de la demande: 28.07.2010
(73) Titulaire: Laboratoire de la Mer, 35400 Saint-Malo (FR)
(72) Inventeur: Beaulieu, Anne, 35800 Dinard (FR)
(74) Mandataire: Cabinet Le Guen Maillet

(56) Documents cités:
- WO-A-2005/099650
- WO-A2-01/78818
- WO-A2-2008/122791
- DE-A1- 19 527 943
- FR-A- 2 854 574
- GB-A- 2 395 129
- US-A- 6 125 843
- US-B1- 6 361 521

## Description

### 1. Domaine de l'invention

L'invention concerne le domaine des pulvérisateurs et/ou des brumisateurs nasaux.

Plus précisément, l'invention concerne une canule destinée à être montée à l'extrémité d'un flacon contenant une solution, et son procédé de fabrication.

### 2. Art antérieur

Il est connu d'utiliser un pulvérisateur et/ou un brumisateur pour administrer une solution par voie nasale, par exemple pour traiter des complications chez un individu souffrant d'un rhume, ou atteint d'une rhinite allergique, ...

On a ainsi proposé de monter des canules, à l'extrémité d'un récipient ou d'un flacon, contenant une solution fluide thérapeutique, pour faciliter l'administration de la solution. De telles canules se présentent suivant différentes formes, par exemple avec un conduit interne droit ou incliné, et différents modèles. Elles peuvent par ailleurs être équipées ou non d'un embout anti-intrusion. Elles sont en outre actuellement obtenues à partir d'une matière unique, par exemple du polyéthylène (PEHD) ou du polypropylène (PP), et sont donc rigides.

Selon l'application visée, on a également envisagé d'adapter ces canules pour obtenir un débit et une forme du jet prédéterminé.

Un inconvénient de ces canules connues est que l'utilisateur risque d'endommager ses cloisons nasales avec l'extrémité de la canule, s'il fait un faux mouvement. Ce risque est d'autant plus critique si la solution est administrée à un nourrisson ou à un enfant en bas âge.

Un autre inconvénient de ces techniques de canule connues est qu'elles ne s'adaptent pas à la morphologie de certains utilisateurs.

Différentes canules nasales connues de l'art antérieur sont divulguées par exemple dans les documents US-B1-6 361 521, GB-A-2 395 129, DE 195 27 943 A1, WO 2005/099650 A, FR-A-2 854 574, US 6 125 843 A, WO 01/78818 A2 et WO 2008/122791 A2.

### 3. Objectifs de l'invention

L'invention a notamment pour objectif de pallier ces différents inconvénients de l'état de l'art.

Plus précisément, l'invention a pour objectif d'améliorer les aspects de sécurité liés à l'utilisation d'un brumisateur ou d'un pulvérisateur nasal.

Un autre objectif de l'invention est d'améliorer le confort de l'utilisateur.

### 4. Exposé de l'invention

Ces objectifs, ainsi que d'autres qui apparaîtront par la suite, sont atteints à l'aide d'une canule pour l'administration d'une solution fluide dans une narine.

Selon l'invention, une telle canule comprend deux portions rigides, une première portion rigide formant une portion de liaison avec un récipient, une deuxième portion rigide étant destinée à être introduite dans ladite narine, et une portion souple disposée entre ladite première portion rigide et ladite deuxième portion rigide, une partie substantielle de ladite portion souple étant conformée de façon à présenter une surface extérieure antidérapante, ladite portion souple étant solidaire de ladite première portion rigide et ladite portion souple recouvrant partiellement la partie supérieure de ladite première portion rigide.

Ainsi, l'invention propose une nouvelle approche des canules convenant aux brumisateurs ou aux pulvérisateurs de solutions nasales, d'un usage particulièrement simple et sécurisant pour l'utilisateur.

Selon un aspect particulier de l'invention, la première portion rigide est une portion de liaison avec un récipient.

Ainsi, le montage de la canule sur le récipient, ou sur le flacon, est le plus souvent rapide, sans déformation de la portion de liaison qui pourrait, dans certains cas, nuire à l'efficacité du montage.

Selon un autre aspect particulier de l'invention, ladite portion souple est disposée entre ladite première portion de liaison et ladite deuxième portion rigide.

Ainsi, on peut profiter de la souplesse de la portion souple pour modifier la position relative des deux portions rigides et l'adapter à la morphologie de l'utilisateur ou de l'individu à laquelle la solution est administrée.

De façon avantageuse, ladite portion souple présente une collerette destinée à empêcher ou limiter la pénétration de ladite canule dans ladite narine.

On limite ainsi les risques de lésions ou d'irritation des parois nasales.

Dans au moins un mode de réalisation particulier de l'invention, lesdites portions rigides sont en polyéthylène ou en polypropylène et ladite portion souple est en matière plastique souple ou en caoutchouc.

Selon un autre aspect particulier de l'invention, une partie substantielle de ladite portion souple est conformée de façon à présenter une surface extérieure antidérapante.

Ainsi, on réduit les risques de glissement de la canule sur la lèvre supérieure sur laquelle elle peut prendre appui, pouvant occasionner des lésions ou des ecchymoses à l'utilisateur.

De façon avantageuse, ladite portion souple est inclinée par rapport à ladite première portion rigide.

Ainsi, la canule forme un bec à l'extrémité du flacon, permettant d'introduire l'extrémité de la canule dans la narine tout en tenant le flacon retourné.

L'invention concerne également un procédé de fabrication d'une canule pour l'administration d'une solution fluide dans une narine telle que décrite précédemment comprenant une étape de co-injection des matières constitutives des portions rigides et de la portion souple dans un même moule, laquelle étape comprend les étapes suivantes:
- une première étape, au cours de laquelle on obtient la première portion rigide de liaison avec le récipient, par injection de sa matière dans le moule;
- une deuxième étape d'obtention de la deuxième portion rigide, par injection de la matière de la deuxième partie rigide;
- une troisième étape, au cours de laquelle on solidarise la portion souple à chacune des portions rigides en injectant la matière de la partie souple dans le moule.

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :
- la figure 1A est une vue schématique d'un mode de réalisation d'une canule selon l'invention, représenté de face ;
- la figure 1B est une représentation en coupe de la canule selon la portion souple de la canule représentée figure 1A ;
- la figure 1C est une vue de détail AA de la zone supérieure de la canule présentée figure 1A :
- la figure 2 est une vue de côté en perspective de la canule présentée sur la figure 1A ;
- la figure 3 est une vue de dessous de la canule présentée sur la figure 1A ;
- la figure 4 est une vue de dos de la canule présentée sur la figure 1A ;
- la figure 5 est une vue de dessus de la canule présentée sur la figure 1A ;
- la figure 6 est un schéma synoptique des étapes d'un procédé selon l'invention.

### 6. Description détaillée d'un mode de réalisation

### 6.1. Rappel du principe de l'invention

Comme évoqué précédemment, l'invention propose une nouvelle approche des canules pour brumisateur ou pulvérisateur nasal.

Le principe général de l'invention repose plus précisément sur la mise en oeuvre d'une portion souple associée à deux portions rigides pour former une canule.

Une telle canule selon l'invention est par ailleurs réalisée en employant une technologie de co-injection dans un même moule.

### 6.2. Exemple de mode de réalisation de l'invention

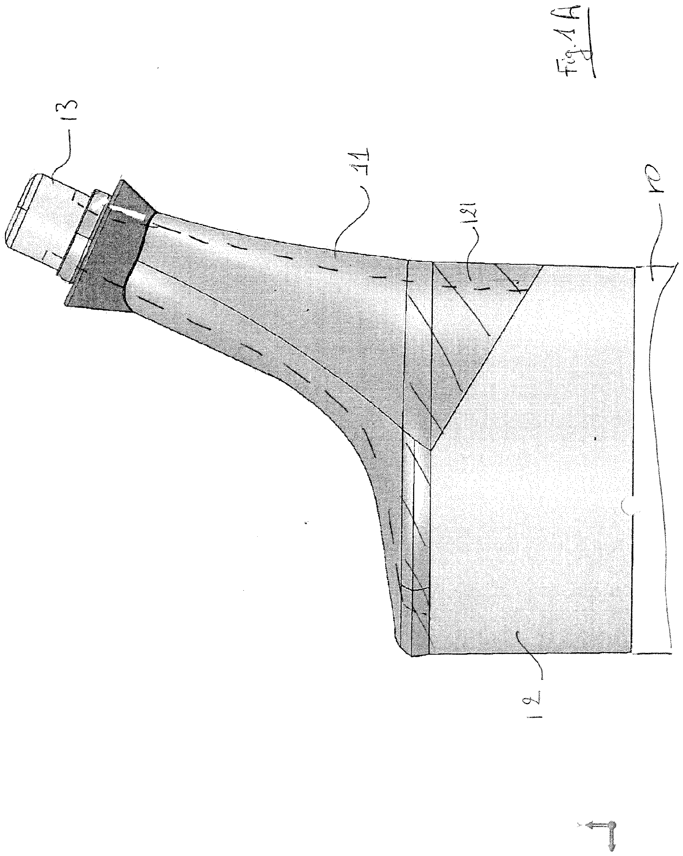

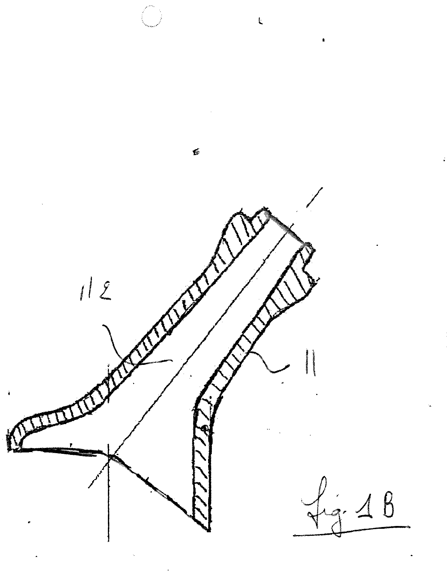

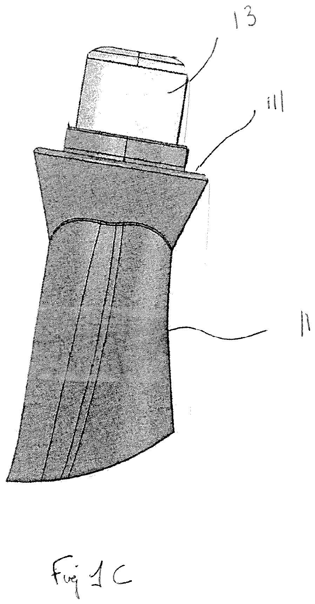

On présente en relation avec la figure 1 un exemple de mode de réalisation de l'invention dans lequel une canule est vissée sur le col 10 d'un flacon destinée à contenir une solution nasale à base d'eau de mer.

Dans des variantes de ce mode de réalisation, il peut également être envisagé de solidariser la canule au flacon en mettant en oeuvre d'autres techniques connues appropriées, telles que par exemple le surmoulage, le collage, le clippage, le sertissage, ou le couplage par baillonnette, ....

Comme on peut le voir sur la figure 1A, la canule bi-matière selon l'invention est ainsi constituée d'une portion souple 11 centrale souple et inclinée, solidaire d'une portion rigide formant support 12, reliant la canule au flacon, qu'elle recouvre partiellement dans sa partie supérieure 121 (représentée avec des hachures sur la figure 1A) et d'une deuxième portion rigide 13 destinée à être introduite dans la narine de l'utilisateur.

Dans le cadre de l'invention, on entend par le terme bi-matière la coexistence de deux matières différentes sur au moins une partie de la canule.

Dans ce mode de réalisation de l'invention les portions rigides 12 et 13 sont en polypropylène et la portion souple 11 est en élastomère et est pourvue d'une surface extérieure antidérapante, par exemple du fait de la présence de petits picots sur cette surface.

Comme on peut le voir sur la figure 1B, la portion souple présente un canal central 112 permettant de laisser passer la solution liquide.

La portion souple 11 présente une collerette 111 (voir figure 1C) destinée à empêcher ou limiter la pénétration de la canule dans la narine.

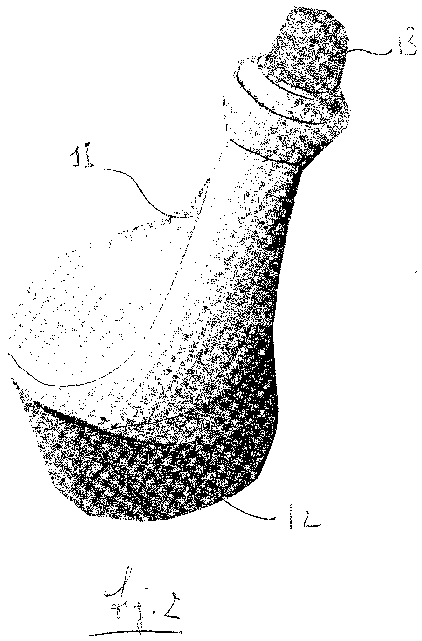

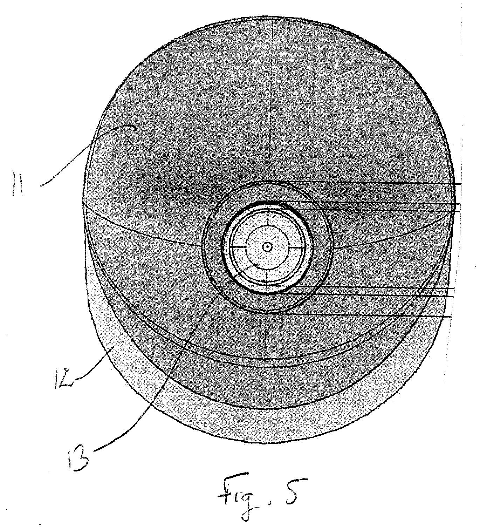

La forme extérieure de la canule est présentée respectivement vue de côté de dessous, de dos et de dessus, sur les figures 2, 3, 4 et 5.

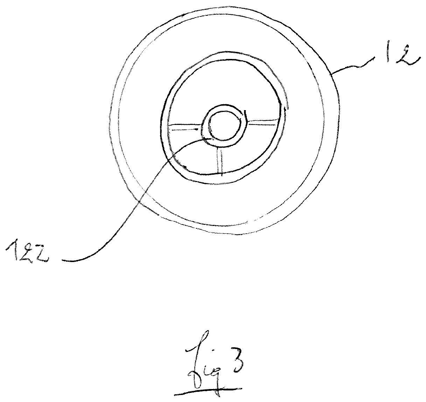

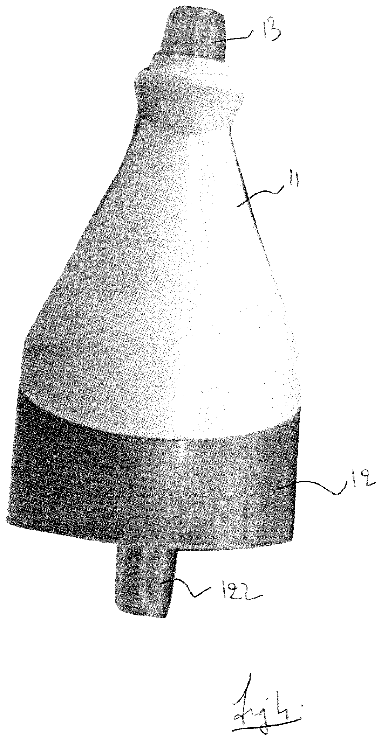

Comme on peut le voir sur les figures 3 et 4, la partie rigide 12 comprend une partie tubulaire 122 faisant saillie vers le bas, destiné à pénétrer dans le flacon.

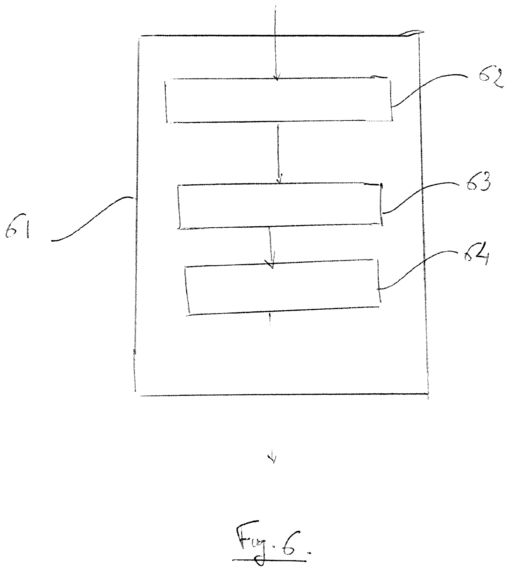

On présente en référence à la figure 6, sous forme de schéma synoptique, les étapes d'un procédé de fabrication de la canule présentée sur les figures précédentes.

Ce procédé comprend une étape 61 de co-injection des matières constitutives des portions rigides et de la portion souple dans un même moule.

L'étape 61 comprend :
- une première étape 62, au cours de laquelle on obtient la portion de liaison avec le flacon, par injection de sa matière dans le moule ;
- une deuxième étape 63 d'obtention de la deuxième partie rigide, par injection de la matière de la deuxième partie rigide ;
- une troisième étape 64, au cours de laquelle on solidarise la portion souple à chacune des portions rigides en injectant la matière de la partie souple dans le moule.

Dans une variante de ce procédé, il peut également être prévu de réaliser les deux portions rigides de la canule au cours d'une même étape.

Il convient de noter que le procédé mis en oeuvre pour fabriquer la canule, et l'emploi d'une matière souple pour obtenir la portion souple, procure ainsi à la canule un effet moins traumatisant pour l'utilisateur lors de l'application du brumisateur.

### 6.3. Autres caractéristiques et avantages de l'invention

Dans au moins un autre mode de réalisation de l'invention, il peut également être envisagé de ménager dans la canule un canal interne rectiligne aligné avec les portions rigides et/ou la portion souple.

## Revendications

1. Canule pour l'administration d'une solution fluide dans une narine, comprenant deux portions rigides (12, 13), une première portion rigide (12) formant une portion de liaison avec un récipient, une deuxième portion rigide (13) étant destinée à être introduite dans ladite narine, et une portion souple (11) disposée entre ladite première portion rigide (12) et ladite deuxième portion rigide (13), **caractérisée** en qu'une partie substantielle de ladite portion souple (11) est conformée de façon à présenter une surface extérieure antidérapante, et en ce que ladite portion souple (11) est solidaire de ladite première portion rigide (12) et recouvre partiellement la partie supérieure (121) de ladite première portion rigide (12).

2. Canule selon la revendication 1, **caractérisée en ce que** ladite portion souple (11) présente une collerette (111) destinée à empêcher ou limiter la pénétration de ladite canule dans ladite narine.

3. Canule selon l'une quelconque des revendications 1 à 2, **caractérisé en ce que** lesdites portions rigides (12, 13) sont en polyéthylène ou en polypropylène et ladite portion souple (11) est en matière plastique souple ou en caoutchouc.

4. Canule selon l'une quelconque des revendications 1 à 3, **caractérisé en ce que** ladite portion souple (11) est inclinée par rapport à ladite première portion rigide (12).

5. Procédé de fabrication d'une canule pour l'administration d'une solution fluide dans une narine selon l'une des revendications 1 à 4, **caractérisé en ce qu'**il comprend une étape (61) de co-injection des matières constitutives des portions rigides (12, 13) et de la portion souple (11) dans un même moule, laquelle étape (61) comprend les étapes suivantes:
une première étape (62), au cours de laquelle on obtient la première portion rigide (12) de liaison avec le récipient, par injection de sa matière dans le moule;
une deuxième étape (63) d'obtention de la deuxième portion rigide (13), par injection de la matière de la deuxième partie rigide;
une troisième étape (64), au cours de laquelle on solidarise la portion souple (11) à chacune des portions rigides (12, 13) en injectant la matière de la partie souple dans le moule.

## Patentansprüche

1. Kanüle zur Verabreichung einer flüssigen Lösung in ein Nasenloch, umfassend zwei starre Abschnitte (12, 13), wobei ein erster starrer Abschnitt (12) einen Verbindungsabschnitt mit einem Behälter bildet und ein zweiter starrer Abschnitt (13) dazu bestimmt ist, in das Nasenloch eingeführt zu werden, sowie einen weichen Abschnitt (11), der zwischen dem ersten starren Abschnitt (12) und dem zweiten starren Abschnitt (13) angeordnet ist, **dadurch gekennzeichnet, dass** ein wesentlicher Teil des weichen Abschnitts (11) so ausgebildet ist, dass er eine rutschhemmende Außenfläche aufweist und dass der weiche Abschnitt (11) fest mit dem ersten starren Abschnitt (12) verbunden ist und den oberen Teil (121) des ersten starren Abschnitts (12) teilweise abdeckt.

2. Kanüle nach Anspruch 1, **dadurch gekennzeichnet, dass** der weiche Abschnitt (11) einen Kragenrand (111) aufweist, der dazu bestimmt ist, das Eindringen der Kanüle in das Nasenloch zu verhindern oder zu begrenzen.

3. Kanüle nach einem der Ansprüche 1 bis 2, **dadurch gekennzeichnet, dass** die starren Abschnitte (12, 13) aus Polyethylen oder aus Polypropylen sind und der weiche Abschnitt (11) aus weichem Kunststoff oder aus Kautschuk ist.

4. Kanüle nach einem der Ansprüche 1 bis 3, **dadurch gekennzeichnet, dass** der weiche Abschnitt (11) bezüglich des ersten starren Abschnitts (12) geneigt ist.

5. Verfahren zur Herstellung einer Kanüle zur Verabreichung einer flüssigen Lösung in ein Nasenloch nach einem der Ansprüche 1 bis 4, **dadurch gekennzeichnet, dass** es einen Schritt (61) der Co-Injektion der Bestandteile der starren Abschnitte (12, 13) und des weichen Abschnitts (11) in ein und dieselbe Form umfasst, wobei der Schritt (61) die folgenden Schritte umfasst:
einen ersten Schritt (62), um Laufe dessen der erste starre Verbindungsabschnitt (12) mit dem Behälter durch Einspritzen seines Werkstoffs in die Form erhalten wird,
einen zweiten Schritt (63) des Erhaltens des zweiten starren Abschnitts (13) durch Einspritzen des Werkstoffs des zweiten starren Teils,
einen dritten Schritt (64), im Laufe dessen der weiche Abschnitt (11) jeweils fest mit den starren Abschnitten (12, 13) verbunden wird, indem der Werkstoff des weichen Teils in die Form gespritzt wird.

## Claims

1. Nozzle for the administration of a liquid solution in a nostril, comprising two rigid portions (12, 13), a first rigid portion (12) forming a portion linking with a receptacle, a second rigid portion (13) being intended to be inserted into said nostril, and a flexible portion (11) arranged between said first rigid portion (12) and said second rigid portion (13), **characterized in that** a substantial part of said flexible portion (11) is formed in such a manner as to have a non-slip exterior surface, and **in that** said flexible portion (11) is integral with said first rigid portion (12) and partially covers the upper part (121) of said first rigid portion (12).

2. Nozzle according to Claim 1, **characterized in that** said flexible portion (11) has a collar (111) intended to prevent or to limit the penetration of said nozzle in said nostril.

3. Nozzle according to either of Claims 1 and 2, **characterized in that** said rigid portions (12, 13) are made from polyethylene or from polypropylene and said flexible portion (11) is made from flexible plastics or from rubber.

4. Nozzle according to any one of Claims 1 to 3, **characterized in that** said flexible portion (11) is inclined relative to said first rigid portion (12).

5. Method for manufacturing a nozzle for the administration of a liquid solution in a nostril according to one of Claims 1 to 4, **characterized in that** it comprises a step (61) of co-injection of the constituent materials of the rigid portions (12, 13) and of the flexible portion (11) in one and the same mould, which step (61) comprises the following steps:
a first step (62), during which the first rigid portion (12) linking with the receptacle is obtained by injection of the material thereof into the mould;
a second step (63) of obtaining the second rigid portion (13) by injection of the material of the second rigid part;
a third step (64) during which the flexible portion (11) is secured to each of the rigid portions (12, 13) by injecting the material of the flexible part into the mould.
